# EUROPEAN PATENT APPLICATION

(11) **EP 1 203 588 A1**
(43) Date of publication of application: **08.05.2002**
(21) Application number: 00123451.7
(22) Date of filing: 06.11.2000
(51) Int. Cl.: A61K 38/19

(54) **Sterol-independent regulation of ABC1 promoter via oncostatinM**

(71) Applicant: BAYER AG, 51368 Leverkusen (DE)
(72) Inventor: Schmitz, Gerd, Prof.Dr., 93161 Sinzing (DE); Porsch-Özcürümez, Mustafa, Dr., 93164 Laaber (DE); Langmann, Thomas, Dr., 93057 Regensburg (DE); Dittrich-Wengenroth, Elke, Dr., 42109 Wuppertal (DE)

(57) **Abstract**

The ATP-binding cassette transporter-1 (ABC1) was recently identified as the defective gene in genetic high density lipoprotein (HDL) -deficiency syndromes. These are characterized by a defect in cellular cholesterol removel through the Golgi pathway and the almost complete absence of plasma HDL cholesterol. ABC1 shows a differentiation-, cAMP- and sterol dependent upregulation in human monocytes. The object of the present invention was to identify non-cholesterol derivatives as mediators of ABC1 gene expression.

## Description

The ATP-binding cassette transporter-1 (ABC1) was recently identified as the defective gene in genetic high density lipoprotein (HDL) -deficiency syndromes. These are characterized by a defect in cellular cholesterol removel through the Golgi pathway and the almost complete absence of plasma HDL cholesterol. ABC1 shows a differentiation-, cAMP- and sterol dependent upregulation in human monocytes. The object of the present invention was to identify non-cholesterol derivatives as mediators of ABC1 gene expression in particular those mediators which upregulate the expression preferably in the liver.

Epidemiological studies have demonstrated that plasma HDL cholesterol (HDL-C) concentrations are inversely related to the incidence of coronary artery disease. Thereby genetic factors have a key role in regulating HDL levels. One rare form of genetic HDL deficiency is an autosomal recessive disorder, tangier disease (TD), with almost complete absence of HDL-C levels. TD patients accumulate cholesterol esters, resulting in enlarged yellow tonsils, hepatosplenomegaly, peripheral neuropathy and deposits in the rectal mucosa.

Using a graphical linkage exclusion strategy, WO 000 18912 assigned the TD locus to chromosome 9q31. Rust et al. (1) came to the same conclusion. One year later several groups independently identified the TD gene to be ABC1 (2-4).

Human ABC1 has been cloned in 1999 (accession no. AJ012376)(5). It is an integral membrane binding protein (MG 226 kD) which contains two ATP-binding segments and two transmembrane sections, each encoding six transmembrane helices. ABC1 is highly expressed in placenta, liver, lung, adrenal glands and fetal tissue (5). The protein itself has a consensus sequence in its regulatory domain for phosphorylation by cAMP-dependent protein kinases, suggesting that direct phosphorylation of this protein may have functional significance (7).

At the transcriptional level ABC1 is modulated by cellular cholesterol stores (5, 6). Recently, numerous SP1 binding sites and putative sterol regulatory elements have been characterized within the ABC1 promoter region (8, 12). Moreover a LXR/RXR binding site has been identified (9). Further reference is made to Gen Bank AJ 252277.

Due to its regulation and substrate profil ABC1 has been associated with the initial steps of reverse cholesterol transport (5). Mutations in ABC1 severely impair apolipoprotein-mediated lipid efflux from cells (6).

Lipid profiles from ABC1 knockout mice (-/-) revealed an ∼ 70% reduction in cholesterol, markedly reduced plasma phopholipids, and an almost complete lack of high density lipoproteins (HDL) and apolipoprotein AI when compared with wild type littermates (+/+). Low density cholesterol (LDL) and apolipoprotein B were also significantly reduced (10). Moreover, inactivation of ABC1 gene led to an increase in the absorption of cholesterol in mice fed a high-fat and -cholesterol diet.

In the present study we analyzed the ABC1 promoter for the modulation of sterol-independent gene expression. An up-regulation of ABC1 promoter via oncostatinM could be shown in HuH cells (human hepatoma).

### METHODS

### Genomic and cDNA Cloning

Standard molecular-biological procedures were carried out as described (11). Primers for the amplification of the putative ABC1 promoter sequence were based on a genomic sequence provided by the Whitehead Institute/ MIT Center for genome Research, Cambridge, MA, USA (acc. no. AC012230). The human BAC clone RP11-1M10 which contains the genomic ABC1 5' region was provided by the Resource Center of the German Human Genome Project (RZPD) and served as a template for the amplification of the promoter sequence with the High Fidelity PCR System (Boehringer Mannheim).

The obtained PCR fragments were cloned into pCR 2.1 TOPO vectors (Invitrogen) and sequenced on an automated fluorescence DNA sequencer using the ALFexpress AutoRead sequencing kit (Pharmacia).

The reporter construct of the ABC1 promoter sequence was cloned by ligation of PCR fragments into the Bgl II and Nhe I restriction sites of the pGL3-basic vector. A 1.1 kb promoter fragment was obtained by PCR using the reverse primer A1-LUCIR 5'-GGGAGATCTTTTCCACTTTTGTGTTTGCGTCTC-3' (position +224) in combination with the forward primer A1-LUC6
5'-GGGGCTAGCTAAGTTGGAGGTCTGGAGTGGCTA-3' (position -919).

### Cell Culture and Transfection

Human hepatoma cells (HuH) were cultured in RMPI medium (GIBCO), supplemented with 10% fetal calf serum, glutamine and antibiotics (penicillin and streptomycin) (all from GIBCO) according to standard protocols. Cells were incubated in a 5% CO₂ atmosphere at 37°C.

OncostatinM (OSM; R & D) stimulation experiments Were performed as follows. 2 x 10³ cells were seeded in 96-well plates with 100 µl medium (s.o.) containing 10% FCS. After 24 hours cells were transiently transfected and incubated for another 24 hours. Transfection with 10 ng ABC1 promoter vector plus 20 ng promoterless pGL3-basic vector was performed with FuGENE6 (ROCHE) as described by the manufacturer. A pGL3-control vector which contains the SV40 early promoter was used as a positive control. After addition of increasing amounts (0,1; 1; 10 nM) of OSM in OPTI-MEM I media cells were incubated for the indicated time points before measurement of luciferase activity.

### Luciferase Assay

After different stimulation periods cells were lysed in reporter lysis buffer (Promega) and luciferase assay reagent (Promega) containing luciferyl-CoA was added. Luciferase activity was determined in a camera system (developed by BAYER). Each experiment was repeated two to three times and measurements were done in triplicate.

### RESULTS

HuH cells were transfected as described under materials and methods. 24h after transfection cells were incubated with increasing amounts (0; 0,1; 1; 10 nM) of OSM for 2, 4, 8 and 24h, respectively. A dose-dependent induction of ABC1 promoter could be shown. Maximal activation (8-fold) was reached within 24h with 10 nM OSM. However, already after 4h a clear increase of gene expression (5- and 6-fold) could be measured with 1 and 10 nM of OSM, respectively. The effective concentration for half-maximum ABC1 promoter activation lies in the range of 0,1nM.

These results show an efficient cholesterol-independent induction of ABC1 expression in human liver cells by the pro-inflammatory cytokine OSM.

### LITERATURE

- 1.: Rust S. et al. Assignment of Tangier disease to chromosome 9q31 by agraphical linkage exclusion strategy. Nature Genet. 20, 96-98 (1998)
- 2.: Bodzioch M. et al. The gene encoding ATP-binding cassette transporter 1 is mutated in Tangier disease. Nature Genet. 22, 347-351 (1999)
- 3.: Brooks-Wilson A. et.al. Mutations in ABC1 in Tangier disease and familial high-density lipoprotein deficiency. Nature Genet. 22, 336-345 (1999)
- 4.: Rust S. et. al. Tangier disease is caused by mutations in the gene encoding ATP-binding cassette transporter 1. Nature Genet. 22, 352-355 (1999)
- 5.: Langmann T. et.al. Molecular cloning of the human ATP-binding cassette transporter 1 (hABC1): Evidence for sterol-dependent regulation in macrophages. Biochem. Biophys. Res. Commun. 258, 73-76 (1999)
- 6.: Lawn RM. et. al. The tangier disease gene product ABC1 controls the cellular apolipoprotein-mediated lipid removal pathway. J. Clin. Invest. 104, R25-R31 (1999)
- 7.: Becq F. et. al. ABC1, an ATP binding cassette transporter required for phogocytosis of apoptotic cells, generates a regulated anion efflux after expression in Xenopus laevis oocytes. J. Biol. Chem. 272, 2695-2699 (1997)
- 8.: Pullinger CR et. al. Analysis of hABC1 gene 5'end: Additional peptide sequence, promoter region and four polymorphisms. Biochem. Biophys. Res. Commun. 271, 451-455 (2000)
- 9.: Costet P. et. al. Sterol-dependent transactivation of the ABC1 promoter by LXR/RXR. J. Biol. Chem. 275, 28240-28245 (2000)
- 10.: McNeish J et. al. High density lipoprotein deficiency and foam cell accumulation in mice with targeted disruption od ATP-binding cassettte transporter-1. Proc. Natl. Acad. Sci. USA 97, 4245-4250 (2000)
- 11.: Sambrook J. et. al. Molecular Cloning: A Laboratory Manual. Cold Spring Harbor, New York, Cold Spring Harbor Laboratory Press, 1989
- 12.: Santamarina-Fojo S. et. al. Complete genomic sequence of the human ABCA1 gene: Analysis of the human and mouse ATP-binding casette A promoter Proc. Natl. Acad. Sci. USA 97, 7987-7992 (2000).

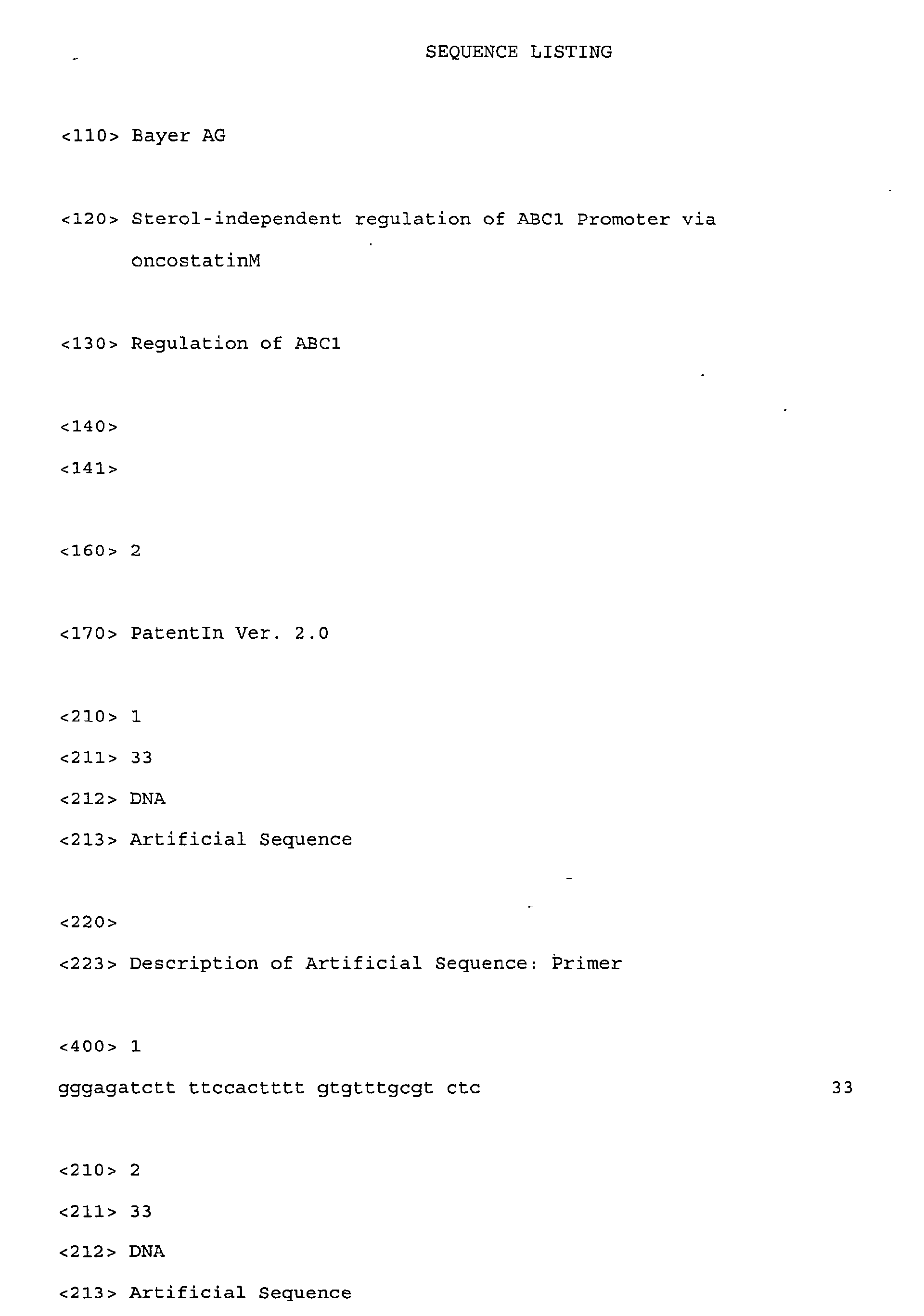

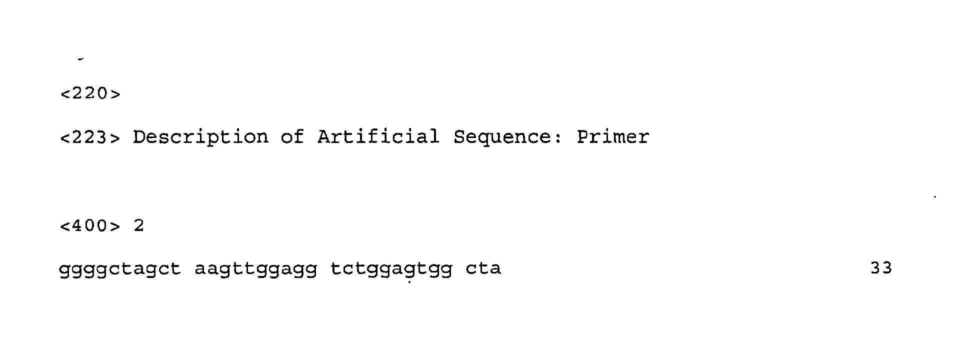

## Claims

1. A medicament containing non-cholesterol derivatives as mediators of ABC-1 gene expression.

2. A medicament according to claim 1 wherein the mediator is oncostatinM.
